# EUROPEAN PATENT APPLICATION

(11) **EP 4 104 852 A1**
(43) Date of publication of application: **21.12.2022**
(21) Application number: 22179326.8
(22) Date of filing: 15.06.2022
(51) Int. Cl.: A61K 38/40, A61K 33/34, A61K 33/30, A61K 33/04, A61K 31/375, A61K 31/593, A23L 33/00, A61K 45/06, A61P 31/04, A61P 31/10, A61P 31/14, A61P 31/16

(54) **COMPOSITION WITH IMMUNOSTIMULATING AND IMMUNOREGULATING ACTIVITY TO PROMOTE THE IMMUNE RESPONSE OF THE HUMAN ORGANISM**

(30) Priority: 16.06.2021 IT 202100015791
(71) Applicant: Aqma Italia S.p.A., 20123 Milano (IT)
(72) Inventor: PIRONTI, Michele, 80056 ERCOLANO (IT)
(74) Representative: Coppo, Alessandro

(57) **Abstract**

The present invention concerns an immunostimulating composition containing a combination of lactoferrin with vitamins C and D, in particular D3, and a micronutrient selected from zinc, copper, selenium, preferably a mixture thereof. The invention also relates to a food supplement containing the composition and excipients to modulate the body's immune response and reduce and/or treat infections of bacterial, fungal, or viral origin.

## Description

### FIELD OF THE INVENTION

The present invention relates to a composition with immunostimulating and immunoregulatory activity to promote an immune response in the human body.

The present invention originates in the pharmaceutical, nutraceutical, and food supplement sectors.

Specifically, the present invention relates to a food supplement containing a combination of biologically active ingredients that exert a synergistic action of stimulation and modulation of the body's immune response against pathogens such as bacteria, viruses, fungi, parasites, and other pathogenic organisms.

### STATE OF THE ART

In the human body, the immune response consists of two components: innate response and specific or adaptive response, also known as acquired immunity.

The innate response represents the first non-specific and rapid response to a pathogenic microorganism or antigen.

The components of the innate immune response include the body's physical barriers, such as the skin and mucous membranes, and the cells of the immune system such as neutrophils, macrophages, monocytes, and soluble factors such as cytokines and complement.

The specific immunity or adaptive response, on the other hand, represents the set of responses of specific type activated by the immune system against specific antigens. The activation of this type of response takes longer to activate the various components that interact with the antigen and is essentially based on the activation of B and T lymphocytes, cells specialized in immune functions, and is characterized by the important specificity of the receptors involved, such as antibodies in the case of B lymphocytes and the T cell receptor in the case of T lymphocytes.

Acquired or adaptive immunity can be acquired naturally when the organism comes into contact with an antigen or when the immune system retains the memory of diseases it has already had, the so-called active natural acquired immunity, or artificially acquired immunity when a vaccine containing a specific antigen is administered. Adaptive immunity works through humoral immunity, by blood route and cell-mediated immunity.

In the first case, the B lymphocytes, which are activated to produce antibodies suitable for eradicating infectious agents, are involved.

In the second case, the T lymphocytes act, which are activated and secrete some inflammatory molecules such as cytokines.

The production of these lymphocytes, as a consequence of exposure for the first time to an antigen, is called primary immune response.

When the organism is subsequently exposed to the same antigen, a secondary immune response is determined thanks to the acquired memory. This response is faster and more effective and represents one of the main characteristics of specific immunity. Among the therapeutic means available to improve the body's immune response there are vaccines and immunostimulants, which are almost always also immunoregulatory. Vaccines act by activating the secondary immune response and are therefore highly specific for the antigen. They are therefore highly effective to prevent or reduce the symptoms of a specific infection but do not exert any general immunostimulating action. Immunostimulants solicit the immune system in a non-specific way, increasing the body's innate ability to fight infections.

The present invention originates in the sector of immunostimulating substances. These may be divided into two main groups: immunostimulating drugs, generally of synthetic or biotechnological origin, and immunostimulants of natural origin.

In recent years, the interest in the latter class of immunostimulants has grown considerably since, while improving the body's defenses and immune response, they are easily accessible without a doctor's prescription and have fewer side effects. Although there are a considerable number of products with immunostimulating activity on the market, the recent spread of aggressive viral forms with high transmissibility such as SARS-COVID-19 has made the need to develop products with a high immunostimulating activity and greater specificity against viral infections more evident. One of the objects of the present invention is to provide a composition with an immunostimulating and/or immunoregulatory action on the human immune system which determines a rapid and robust immune response by activating different mechanisms of action of the human organism.

Another object is to provide a food supplement containing a combination of selected components with an immunostimulating action that increase and/or modulate the body's immune response without the side effects that can be found with the use of vaccines or synthetic immunostimulating drugs.

### SUMMARY OF THE INVENTION

The present invention originates from the need to have a supplement combining immune system stimulation properties with an antioxidant and anti-inflammatory action which reduces the inflammatory response that commonly occurs in the case of human body infections.

The inventors have now found that by combining selected biologically active components, an immunostimulating activity of components of the body's immune response is achieved, which mainly intervene in the case of infections, in particular viral infections, for example due to influenza viruses and/or SARS-CoV-2.

As part of the research activity, the inventors have observed that the combination of lactoferrin, with vitamin C, a vitamin D, and a mineral or micronutrient selected from zinc, copper, selenium, and mixtures thereof, produces a robust immunostimulating and/or immunomodulatory action. The immunostimulating activity is increased when the three minerals are present.

Starting from this observation, the Applicant has found that a composition containing lactoferrin, vitamin C and vitamin D combined with specific minerals or such as zinc, copper, selenium is particularly suitable for preventing or treating infections of the organism that cause inflammatory symptoms, as it occurs in the case of viral infections affecting the respiratory airways, e.g. influenza virus, adenovirus, SARS-COV-2 infections.

The combination of active components of the composition described herein activates the non-specific immune response, resulting in a concomitant immunomodulating and anti-inflammatory effect substantially free of the side effects found with the use of traditional drugs.

According to a first aspect, the present invention relates to a composition with immunostimulating activity comprising lactoferrin, vitamins C and D, preferably vitamin D3, in combination with at least one mineral selected from zinc, copper, selenium and mixtures thereof, each mineral optionally being in the form of a salt or oxide.

The combination of the invention finds application in activating the immune system and/or in modulating its response against pathogens such as bacteria, fungi, and viruses.

In one aspect, the object of the invention is a food supplement with immunostimulating activity comprising a combination of lactoferrin, vitamin C, vitamin D with the minerals zinc, copper, selenium, and a physiologically acceptable carrier for use in the prevention and/or treatment of an infection of viral, bacterial, or mixed origin.

In these cases, the antigen triggering the infection is a bacterium or a virus, for example a virus that is transmitted mainly by air and which initially spreads in the respiratory tract.

It has been observed that the compositions or supplements described herein act on the gene expression regulation of the main components of the immune system and in the maturation, differentiation, and response of immune cells.

In the fields of application of the combination composition of the invention, the uses in the treatment of viral and/or bacterial infections of the airways are preferred, particularly where phlogosis, inflammation, is present.

Typically, the biologically active components contained in the composition of the invention are present in a pharmaceutically and/or nutritionally effective amount.

According to some embodiments, the composition of the invention is a nutraceutical, a food or dietary supplement that can be introduced into the diet regime of a subject in need of an immunomodulating or immunostimulating action.

### DETAILED DESCRIPTION OF THE INVENTION

In accordance with an aspect of the invention, the Applicant has found that the combination of the biologically active components lactoferrin, vitamin C, vitamin D, in particular D3, with a mineral selected from zinc, copper, selenium and mixtures thereof, exerts a high immunostimulating and immunomodulatory actions.

Consequently, the composition described herein finds application in the prevention and/or treatment of infectious diseases, for example viral infections, in particular respiratory infections in particular those transmitted by air, such as COVID-19. Advantageously, in the composition of the invention, the combination of biologically active ingredients and minerals enhances the natural resistance of the human organism in a non-specific way and improves the defenses and the average immune response time, in particular against infections of viral origin.

Furthermore, the combination of biologically active ingredients with the mineral components of the composition described herein acts on the immune system of a subject by reducing the immune response time against infections of bacterial, fungal, or viral origin, improving the symptoms related to the infection and reducing the healing times.

Advantageously, it was observed that the composition and the food supplement described herein also have a stimulating action on innate immunity.

Starting from this observation and the consideration that innate immunity is more pronounced in the population of young age subjects, typically aged 2 to 12 years, and that this young population is less prone to contracting a SARS-COVID infection19 or in any case is less prone to suffer from severe or critical forms of COVID-19 than the adult population, the composition of the invention finds specific use in preventing and/or treating less severe forms of COVID-19. Typically, in these forms, the developed symptoms and general health conditions of the sick subject do not require his hospitalization or his admission to intensive care.

According to a first aspect, a composition as defined in the appended claim 1 is then provided.

According to another aspect, the invention relates to a food supplement comprising an immunostimulating composition containing lactoferrin, a vitamin D, vitamin C in combination with at least one mineral selected from zinc, copper, selenium, and mixtures thereof, each mineral optionally being in the form of a salt or oxide, and a physiologically acceptable excipient.

In another aspect, a chewable tablet as defined in the appended claim 6 is provided. Advantageously, the food supplement described herein in the form of a chewable tablet reduces the absorption time of the biologically active components of the composition and reduces the immune response time.

A basic component of the composition or supplement or chewable tablet disclosed herein is lactoferrin.

The latter is a multifunctional glycoprotein belonging to the transferrin family, secreted by endocrine glands, and contained in neutrophil granulocytes.

Lactoferrin has a molecular mass of 80 KDa, with two binding sites for the ferric ion (Fe³⁺), in analogy with transferrin. Typically, lactoferrin is not saturated with iron and its ferric content varies. The antimicrobial activity of lactoferrin is related to its affinity for Fe³⁺ thanks to its ability to compete in the free state with iron-dependent microorganisms, and to the direct action on the outer membrane of Gram negative bacteria.

Lactoferrin carries out four main activities: it chelates two ferric ions per molecule, interacts with anionic molecules, enters the nucleus, and modulates iron homeostasis. Its ability to chelate two ferric ions per molecule is associated with the inhibition of reactive oxygen species formation and sequestration of iron, a phenomenon that is important in viral and bacterial replication and is the basis of its antiviral and antibacterial activity. The ability to bind to compounds with an anionic surface by virtue of its cationic characteristics is associated with its protective action against viral and bacterial adhesion and towards the entry of these pathogens. Entry into host cells and translocation into the nucleus is related to its ability to modulate iron homeostasis perturbed by the viral infection and related inflammation. In addition, lactoferrin regulates the activation of plasminogen and controls the coagulation cascade, resulting in antithrombotic activity and thus limiting the risks of a frequent complication of SARS-CoV2 infection.

Furthermore, the ability of lactoferrin to bind to viral particles helps inhibit the early stage of viral infections and the post-infection stage.

According to some embodiments, the composition of the invention contains lactoferrin in an amount ranging from 0.1 to 50% by weight, from 1 to 40% by weight, from 5 to 30%, for example 20% by weight.

For example, the composition or supplement described herein may contain from 10 to 1000 mg, from 50 to 500 mg, from 100 to 300 mg of lactoferrin per dosage unit.

The inventors have observed that these effects of lactoferrin are enhanced by the combination with one or more selected minerals such as copper, zinc and selenium. In particular, the combination of lactoferrin in a mixture with the three minerals unexpectedly increases the activities previously described.

The composition of the invention further comprises vitamins C and D.

Vitamin C or the enantiomer (5R)-5-[(1S)-1,2-dihydroxyethyl]-3,4-dihydroxyfuran-2(5H)-one or R-3,4-dihydroxy-5-((S)-1,2-dihydroxyethyl)furan-2(5H)-one, is a substance endowed with a high antioxidant action and antiviral properties, and it is necessary for the physiological functioning of the immune response.

In the composition of the invention, vitamin C may be in the form of ascorbic acid or in the ascorbate salt form, typically salified with an alkaline or alkaline earth metal, for example in the form of sodium, potassium, calcium, magnesium salt, such as magnesium ascorbate, sodium ascorbate, potassium ascorbate or calcium ascorbate, and mixtures thereof. Vitamin C may be in the form of an ester, for example with a fatty acid, for example, palmitic acid or stearic acid such as palmitate or stearate. Advantageously, vitamin C is L-ascorbic acid.

According to some embodiments, vitamin C is in a gastroprotected form resistant to the acidic pH of the stomach, for example protected with a gastro-resistant coating or with a film of a substance resistant to acidic pH, for example at pH from 2 to 5, for example ethylcellulose.

In the human body, vitamin C exerts antiviral properties by exerting a multitude of activities, including stimulation of the lymphocyte activity, regulation of interferon production, modulation of cytokines, and regulation of inflammatory processes and improvement of mitochondrial function and endothelial function. It has also been hypothesized that vitamin C has virucidal properties.

It was observed that integration of vitamin C in the diet has beneficial effects in patients affected by viral infections. In the course of infections, vitamin C plays an important role in the maturation of T cells and in promoting phagocytosis and apoptosis of exhausted neutrophils. In the course of viral infections there is therefore an increased metabolism of vitamin C together with a reduction of ascorbate in the circulation.

Furthermore, the inventors research has led to the identification of how treatment with high doses of vitamin C has proved effective in the treatment of acute respiratory distress syndromes (ARDS).

These indications led the inventors to use vitamin C to formulate the combination composition of the invention.

According to some embodiments, the composition of the invention contains vitamin C in an amount ranging from 0.1 to 50% by weight, from 1 to 40% by weight, from 5 to 30%, for example 20% by weight.

For example, the composition or supplement described herein may contain from 10 to 1000 mg, from 50 to 500 mg, from 100 to 300 mg of vitamin C per dosage unit.

Another vitamin component of the composition is vitamin D. This vitamin, also known as calciferol, comprises a group of fat-soluble secosteroids that exist in different forms of which the most widespread, and of greatest biological interest, are vitamin D2, known as ergocalciferol, and vitamin D3, known as cholecalciferol.

Within the scope of the invention, the use of vitamin D3 with the following formula is preferred:

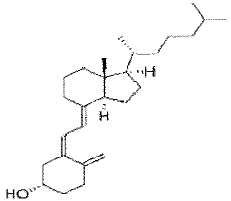

Vitamin D is synthesized from cholesterol following exposure of the skin to UV radiation. In the event of modest sun exposure or reduced dietary intake, the levels of vitamin D in the body may be below physiological values. In these conditions, a higher incidence of infectious diseases of the human organism was observed. Hence the need to provide the body with supplementation to reduce the incidence of viral diseases.

It has been observed that vitamin D promotes the differentiation of monocytes from macrophages, promotes macrophages movement and ability to phagocytosis, the production of superoxide, and bacteria killing by innate immune cells. Furthermore, vitamin D acts on the proper functioning of T cells.

In particular, it has been observed that the vitamin D3 form binds to a specific nuclear receptor known as VDR and, thanks to its immune system regulating action, plays a role as an immunomodulating agent in innate and adaptive immunity.

Based on these activities and the observation that subjects with low vitamin D levels have a higher risk of developing respiratory tract infections, the Applicant tested its immunostimulatory capacity in combination with lactoferrin and vitamin C and its ability to reduce respiratory tract infections. As part of these tests, it was unexpectedly found that the risk of developing respiratory tract infections was reduced when the three vitamins were combined with at least one, preferably all three selected micronutrients or minerals described below.

A suitable micronutrient is zinc, a mineral that plays an important role in the cellular metabolism of immune-adaptive cells and in the development of natural killer cells and neutrophils. Its deficiency causes a reduction in the efficiency of the immune-mediated response, making the human body more susceptible to viral infections.

The administration of the supplement containing zinc described herein increases the number of cytotoxic T lymphocytes and NK cells, reducing the incidence of bacterial or viral infections.

In the composition or supplement, zinc may be present in elemental form, or as an oxide, as a salt, for example zinc chloride, zinc acetate, zinc citrate, or zinc picolinate. According to some embodiments, the composition of the invention contains zinc in an amount ranging from 0.01 to 10% by weight, from 0.1 to 10% by weight, from 0.5 to 5%, for example 1% by weight. Preferably, the zinc is supplied in the form of ZnO. For example, the composition or supplement described herein may contain from 0.1 to 100 mg, from 1 to 50 mg, from 5 to 20 mg of zinc per dosage unit.

Another suitable micronutrient is selenium, a mineral present in trace amounts in the human body, with anti-inflammatory and antioxidant activity.

The Applicant, starting from the observation that a selenium deficiency in the organism is associated with a higher incidence of viral infections and an increased pathogenicity and mutations of viruses, such as poloviruses and murine influenza viruses, has selected selenium as a suitable mineral component of the composition described herein. Selenium may be present in the composition in elemental or salt or ester forms. Preferably, the selenium-based compound is in semi-organic form, for example of selenomethionine or selenium L-methionine.

Selenium plays an important role in regulating the immune response particularly in RNA virus infections where oxidative stress may increase the virus mutation rate and virulence. Its deficiency reduces the innate and acquired immune response, negatively affecting cell-mediated immunity and humoral immunity underlying the production of antibodies.

Furthermore, selenium is involved in the proliferation of T-lymphocytes, and the humoral system, in particular it is active in the production of immunoglobulins. Advantageously, the integration of selenium in the composition in combination with lactoferrin and vitamins C and D improves cell-mediated immunity in subjects with immune deficiency, and also increases the immune response to viruses.

According to some embodiments, the composition of the invention contains selenium or selenium-based compound such as selenium-methionine, in an amount ranging from 0.0001 to 1% by weight, from 0.001 to 0.1% by weight, from 0.005 0.05%, for example 0.01% by weight.

For example, the composition or supplement described herein may contain from 1 to 10000 mcg, from 10 to 1000 mcg, from 50 to 200 mcg of selenium or selenomethionine, for example 100 mcg per dosage unit.

Another suitable micronutrient is copper. This metal-micronutrient is essential for the human body, playing an active role in redox reactions and protein synthesis and a fundamental role for the constitution of the respiratory biological catalyst cytochrome C oxidase, known as complex IV, EC 1.9.3.1.

Copper may be present as a building block of enzymes and transporters that play roles of catalysis and transfer of biological electrons or oxygen in processes that exploit the interconversion of copper type I and II - Cu (I) and Cu (II).

In mitochondria, copper is found in the cytochrome C oxidase enzyme, the last protein in oxidative phosphorylation that binds O₂ between a copper ion and an iron ion, transferring 8 electrons to the O₂ molecule and thereby reducing it to two water molecules, due to the resulting bond with hydrogen.

Copper is also found in many superoxide dismutase enzymes, proteins that catalyze the decomposition of superoxides by converting them, by dismutation, into oxygen and hydrogen peroxide.

The Applicant has observed how copper has intrinsic antimicrobial properties and performs some important functions in the immune response. In particular, it plays a role in the differentiation and proliferation of T cells and in neutrophilic and monocyte macrophages, and also increases the activity of the NK cell.

Furthermore, the Applicant has unexpectedly found that when copper is in combination with selenium and zinc and with lactoferrin and vitamins C and D, the immunostimulating action on the human body is increased.

According to some embodiments, the composition of the invention contains copper or a copper-based compound, such as copper gluconate, in an amount ranging from 0.0003 to 3% by weight, from 0.003 to 0.3% by weight, from 0.010 to 0.1%, for example 0.03% by weight.

For example, the composition or supplement described herein may contain from 5 to 20,000 mcg, from 30 to 3,000 mcg, from 100 to 600 mcg of copper or copper-based compound, for example 300 mcg per dosage unit.

Preferably, the composition described herein does not contain further biologically active ingredients in addition to lactoferrin, ascorbic acid (vitamin C), vitamin D. Unexpectedly, it was observed that the addition of further biologically active components to the composition described herein reduces the immunostimulating activity.

Furthermore, the composition or supplement described herein preferably does not contain oils such as sunflower, corn, linseed oils, and mixtures thereof.

The composition of the invention can be contained in a food supplement together with an edible or physiologically acceptable carrier. The composition or supplement can take a wide variety of preparation forms, according to the desired route of administration. The composition of the invention may be in solid form.

When the composition of the invention is in solid form, it can be in the form of a tablet, capsule, powder, granules, lozenge, or chewable tablet, or to be dissolved in the mouth, and powder or solution/suspension that can be sprayed directly into the oral cavity. It is preferably in the form of a chewable tablet, advantageously with fast release of the active components contained.

The solid form preparations may comprise one or more excipients, such as for example starches, sugars, microcrystalline cellulose and optionally diluents, granulating agents, lubricants, aggregating agents, disintegrating agents.

Typically, the solid form composition may contain an aggregating agent such as tragacanth gum, gum, corn starch or gelatin; excipients such as dicalcium phosphate; a disintegrating agent such as corn starch, potato starch, alginic acid; a lubricant such as magnesium stearate; a sweetening agent such as sucrose, lactose, or saccharin.

If desired, the tablets may be of the chewable type or coated with a film such as gastro-resistant film using traditional techniques.

In some embodiments the composition is a supplement in the form of a capsule, for example in gelatin, which preferably may contain an anti-caking agent, for example mono and diglycerides of fatty acids, Vegetable Magnesium Stearate, Silicon Dioxide and one or more bulking agents for example dibasic calcium phosphate, microcrystalline cellulose.

According to some embodiments, the composition of the invention contains a cellulose-based excipient.

In some embodiments, the composition of the invention further comprises one or more additional components such as additives, fillers, stabilizers, emulsifiers, texturizers, film-forming agents, plasticizers, wetting agents, and thickeners.

In the case of tablets these may be coated with shellac, sugar, or both. To prevent rupture during transit through the upper gastrointestinal tract, the composition may be a formulation with a gastro-resistant or enteric coating.

In the composition or capsules there may also be flavoring agents, preservatives, coloring agents, and the like.

According to some embodiments, the composition is in a form for modulated or controlled release of active ingredients. By way of example, the composition may be a delayed-release, fast-release, or slow-fast-release formulation.

In some embodiments, the composition of the invention comprises, as an excipient, a hydrogenated fatty acid, preferably having a 3 to 20 carbon atoms, 14 to 18 carbon atoms chain. A typical example of a hydrogenated fatty acid is hydrogenated palm oil. In one embodiment, the composition described herein or the food supplement containing it, is in the form of a tablet, for example a chewable tablet containing as carriers or excipients sorbitol, magnesium salts of fatty acids, as an anti-caking agent silicon dioxide. The supplement may contain a natural sweetener, for example sucrose or a synthetic sweetener such as sucralose.

In some embodiments, the composition of the invention may be in a liquid form. When the composition is in a liquid form, it can be in the form of a suspension, emulsion, solution, oral spray, mouthwash. In these cases, the carrier is liquid and may be selected for example from water, glycols, oils, alcohols, and mixtures thereof.

In some embodiments in which the formulation is in a liquid form, for example in the case of a solution, syrup, or elixir, it may contain, in addition to the combination of active ingredients, a sweetening agent such as sucrose and/or methyl and propyl-parabens as preservatives, a coloring agent and a flavoring agent such as cherry or orange flavors.

Suitable compositions may be presented in a single pharmaceutical form and prepared using any of the methods well known in the pharmaceutical or dietary state of the art. In some embodiments, in the compositions of the present invention, the active ingredients are normally formulated in a dosage unit. The dosage unit may contain from 0.1 to 1,000 mg of each active ingredient per dosage unit for a daily administration. A suitable dosage unit is a capsule.

In some embodiments the dose is in the range from 0.001% by weight to about 60% by weight of the formulation.

According to some embodiments, the composition of the invention is a food supplement or a dietary or nutraceutical product.

### Terminology used

As used herein, the term "edible" means edible substances whose use in the formulation of a nutritional or food composition is approved by health authorities.

The term "physiologically acceptable" refers to substances commonly used in the formulation of nutritional, food, or pharmaceutical products. A "physiologically acceptable" carrier can be a pharmaceutically acceptable carrier.

The term "carrier" as used herein indicates a medium, excipient, diluent with which the combination of therapeutic or active ingredients is administered.

Any carrier and/or excipient suitable for the desired form of preparation for administration to humans is contemplated for use with the compounds described in the present invention.

Within the scope of the present application, the terms "micronutrient" and "minerals" are used interchangeably and include iron, copper, selenium as such, in the form of salts or oxides. When used, the term "trace elements" refers to minerals and means that the amounts present in the composition are very low, typically in the order of 10-1000 mcg.

As used herein, the term "combination" means that one or more of the active ingredients are added or mixed with one or other ingredients.

The term combination does not mean that the active ingredients are associated with each other with the formation of chemical bonds or other type of bonds.

The compositions of the invention are suitable for food, nutritional, dietary, or pharmaceutical use in mammals, in particular in humans.

According to some embodiments, the composition of the invention is a nutraceutical, food supplement, a nutritional product, or a dietary product.

In some embodiments, the composition or the supplement containing it are administered once or twice a day.

The following examples are provided to further illustrate some features and embodiments of the present combination composition.

### EXAMPLE 1

Composition with immunostimulating action based on:

| | |
|---|---|
| Lactoferrin | 200 mg |
| Vitamin C | 200 mg |
| Zinc | 10 mg |
| Copper | 300 mcg |
| Selenium | 100 mcg |
| Vitamin D | 50 mcg |
| Excipients | *q.s.* to 1000 mg |

### EXAMPLE 2

Supplement with immunostimulating activity in the form of chewable tablets, having the following qualitative-quantitative composition:

| Active ingredient | mg/mcg |
|---|---|
| Lactoferrin | 200mg |
| Vitamin C | 200 mg |
| Vitamin D3 100 GFP | 20 mg |
| - of which Vitamin D3 | 50 mcg |
| Selenium L-methionine | 19.23 mg |
| - of which selenium | 100 mcg |
| Zinc oxide | 12.5 mg |
| - of which zinc | 10 mg |
| Copper gluconate | 2.3 mg |
| - of which copper | 300 mcg |

Excipients in amounts to reach 1000 mg:
- Sorbitol
- Magnesium salts of fatty acids
- silicon dioxide
- sweetener: sucralose
- orange flavor.

### EXAMPLE 3

Efficacy clinical study of a composition with immunostimulating and/or immunoregulatory activity based on lactoferrin, vitamin D and vitamin C in combination with zinc, copper and selenium in the treatment of respiratory viral infection.

### Aims of the clinical study

The aim of this study is to evaluate the effect of a composition with immunostimulating and/or immunoregulatory activity based on lactoferrin, vitamin D and vitamin C in combination with zinc, copper and selenium, in patients positive for Sars-CoV-2, in addition to the treatment provided by the Italian National Health System (SSN).

### Composition tested in the clinical study

The composition tested was in the form of a tablet for oral administration, in particular in a chewable form. The tablet had the following content of active ingredients (biologically active components): lactoferrin 200 mg, vitamin C 200 mg, vitamin D3 50 mcg (equal to 2000 IU), zinc 10 mg, copper 300 mcg and selenium 100 mcg.

### Population of treated patients

Patients previously followed in a geriatric ward of a hospital were enrolled in the study and followed through remote home assistance in collaboration with their caregivers, in case of positive molecular swab for Sars-Cov-2. 30 patients were enrolled, including elderly subjects, and in case of positive swab the corresponding members of the family unit (mean age 44 + 21 years) affected by Sars-CoV-2, who were treated with a nutraceutical composition based on lactoferrin, vitamin D and vitamin C in combination with zinc, copper, and selenium (Squdo - composition in a tablet form) at a dosage of 1 chewable tablet per day, for 30 days.

All the subjects enrolled continued any therapy already taken for other comorbidities and also took the home protocol provided for covid patients in accordance with the professional and ethical protocols provided by the local health authority. Together with the drugs provided for home treatment for Covid 19, the patients took the composition under study, in the form of a tablet, as a support therapy to evaluate the immunostimulating, anti-inflammatory and antioxidant properties of the combination of active ingredients.

### Parameters and therapeutic treatment

As objective and reproducible parameters, the changes in heart rate and oxygen saturation values in the blood measured by standardized pulse oximeters, measured at time zero (T0) and up to negativization of the molecular buffer (T1) which occurred in an average time of 10.13 + 2.1 days, were considered. The intake of the study composition was continued for up to 30 days, in support of the patient's convalescence even after negativization of the molecular swab. The clinical parameters of the enrolled subjects were statistically processed using the SigmaStat 3.5 software for Windows. The paired T-test was used to analyze the different variables measured before and after the planned treatment, in order to confirm the effectiveness of the therapeutic scheme. Statistical significance was set at P <0.05.

The results recorded for each patient are shown in the following Table 1:

| Patient No. | BPM (T0) | BPM (T1) | SO2 (T0) | SO2 (T1) |
|---|---|---|---|---|
| 1 | 81 | 75 | 94 | 98 |
| 2 | 78 | 70 | 93 | 97 |
| 3 | 75 | 68 | 96 | 99 |
| 4 | 74 | 66 | 92 | 97 |
| 5 | 90 | 83 | 96 | 98 |
| 6 | 75 | 69 | 93 | 99 |
| 7 | 89 | 73 | 95 | 99 |
| 8 | 78 | 75 | 94 | 98 |
| 9 | 82 | 75 | 87 | 97 |
| 10 | 85 | 77 | 96 | 99 |
| 11 | 86 | 78 | 98 | 99 |
| 12 | 74 | 66 | 93 | 98 |
| 13 | 93 | 85 | 92 | 97 |
| 14 | 79 | 74 | 94 | 99 |
| 15 | 76 | 70 | 91 | 98 |
| 16 | 88 | 81 | 95 | 99 |
| 17 | 74 | 68 | 98 | 98 |
| 18 | 78 | 73 | 93 | 98 |
| 19 | 77 | 70 | 92 | 99 |
| 20 | 73 | 69 | 95 | 99 |
| 21 | 78 | 71 | 94 | 98 |
| 22 | 76 | 70 | 93 | 98 |
| 23 | 83 | 74 | 95 | 98 |
| 24 | 89 | 81 | 97 | 99 |
| 25 | 92 | 82 | 94 | 98 |
| 26 | 91 | 81 | 95 | 99 |
| 27 | 96 | 78 | 91 | 98 |
| 28 | 85 | 72 | 92 | 99 |
| 29 | 88 | 74 | 89 | 99 |
| 30 | 83 | 75 | 96 | 99 |

The mean results and the statistical significance values are shown in Table 2:

| (N=30) | BPM (T0) | BPM (T1) | SO2 (T0) | SO2 (T1) | P value |
|---|---|---|---|---|---|
| Mean Value | 82.2 | 74.1 | 93.77 | 98.33 | 0.001 |
| Standard Deviation | 6.49 | 5.04 | 2.36 | 0.69 | 0.001 |

### Results

The results of the study showed a statistically significant improvement in heart rate and oxygen saturation values following continued administration of the treatment, in all 30 patients enrolled. More in detail, there was a statistically significant reduction in heart rate (P< 0.001) with the mean value going from 82.267 + 13.470 bpm at time T0 to the value of 74.667 + 8.845 at time T1. Similarly, there was a statistically significant improvement in the average oxygen saturation values SO2 in the blood, which went from 96.4 ± 1.69 to 98.4 ± 0.76 (P < 0.001). No adverse events were reported during the study.

### Conclusions of the clinical study

In conclusion, the data collected allow to highlight the significant improvement in heart rate and oxygen saturation in patients undergoing treatment with a nutraceutical composition based on lactoferrin, vitamin D and vitamin C in combination with zinc, copper, and selenium (Squdo). At the end of the study, the efficacy and safety of the evaluated therapeutic treatment are confirmed.

## Claims

1. A composition with immunostimulating and/or immunoregulatory activity consisting essentially of lactoferrin, vitamin D, vitamin C in combination with the minerals zinc, copper, selenium, each mineral being optionally in the form of a salt or oxide.

2. The composition with immunostimulating and/or immunoregulatory activity according to claim 1, wherein said vitamin D is vitamin D3.

3. The composition according to claim 1 or 2, wherein the selenium is in the form of selenium L-methionine, the zinc in the form of zinc oxide, the copper in the form of copper gluconate.

4. A food supplement comprising a composition according to any one of claims 1 to 3 and at least one physiologically acceptable carrier or excipient.

5. A chewable tablet comprising a composition according to any one of claims 1 to 3 and at least one physiologically acceptable carrier or excipient.

6. The food supplement according to claim 4 or the chewable tablet according to claim 5 comprising 200 mg lactoferrin, 200 mg vitamin C, 30-50 mcg vitamin D3, 19.23 mcg selenium L-methionine, 12.5 mg zinc oxide and 2.3 mg copper gluconate.

7. The composition according to any one of claims 1-3, or the food supplement according to claim 4 or the tablet according to claim 5 or 6, for use in the prevention or treatment of an infectious disease.

8. The composition or the food supplement for use according to claim 7, wherein the infectious disease is a viral, bacterial and/or fungal infectious disease.

9. The composition or the food supplement for use according to claim 8, wherein the infectious disease is a bacterial infection caused by streptococci, staphylococci, or pneumococci.

10. The composition or the food supplement for use according to claim 7, wherein the infectious disease is a viral infection caused by Adenovirus, Rhinovirus, flu virus, SARS-CoV-2.

11. The composition or the food supplement for use according to claim 7, wherein the infectious disease is a viral or bacterial respiratory infection, in particular of the upper airways.
